Europäisches Patentamt

(19) European Patent Office

Office européen des brevets

(11) Publication number : **0 424 043 B1**

(12) **EUROPEAN PATENT SPECIFICATION**

(45) Date of publication of patent specification :
26.05.93 Bulletin 93/21

(51) Int. Cl.⁵ : **A61K 47/36, A61K 9/06**

(21) Application number : **90311208.4**

(22) Date of filing : **12.10.90**

(54) **Liquid aqueous ophthalmic composition undergoing liquid-gel phase transition.**

(30) Priority : **17.10.89 FR 8913534**

(43) Date of publication of application :
**24.04.91 Bulletin 91/17**

(45) Publication of the grant of the patent :
**26.05.93 Bulletin 93/21**

(84) Designated Contracting States :
**AT BE CH DE DK ES FR GB IT LI LU NL SE**

(56) References cited :
**EP-A- 0 125 759**
**WO-A-88/10121**
**FR-A- 2 588 189**
**US-A- 4 136 173**
**US-A- 4 717 713**
**The Merck Index, 11th ed., Merck & Co. Inc., 1989, pp. 284-285**

(73) Proprietor : **LABORATOIRES MERCK, SHARP & DOHME-CHIBRET**
**3, Avenue Hoche**
**F-75008 Paris (FR)**

(72) Inventor : **Chastaing, Gilles**
**111 Bd Gambetta**
**F-63400 Chamalieres (FR)**

(74) Representative : **Cole, William Gwyn**
**European Patent Department, Merck & Co., Inc., Terlings Park, Eastwick Road**
**Harlow, Essex CM20 2QR (GB)**

## Description

The present invention relates to a liquid ophthalmic composition undergoing liquid-gel phase transition.

In many known ophthalmic compositions, a large proportion of the active principle administered in the eye is removed by lachrymal drainage. The effective therapeutic action of such compositions is consequently limited to a very short time. To overcome this disadvantage, the use of compositions of the gel, solid implant or ointment type have been proposed. However, these dosage forms find patient resistance; liquid forms are better tolerated.

Thus, in order to solve this problem, liquid dosage forms which gel in situ through the effect of a pH change (US 4,136,173) or a temperature rise (US 4,188,373, US 4,474,751) have been proposed.

The major drawback of the above formulations is, in the first case, a non-physiological and hence poorly tolerated pH value, and in the second case, the risk of the formulation gelling before its administration, that is to say on storage, through the effect of a temperature rise.

The object of the present invention is to propose an ophthalmic composition having a sustained activity over a period of time and not possessing these drawbacks.

More specifically, the present invention relates to a liquid ophthalmic composition which undergoes liquid-gel phase transition upon administration to the eye and comprises an aqueous solution of at least one active principle, characterized in that the composition contains 0.1 to 5% by weight of a sulphated polysaccharide derivative selected from kappacarrageenan, iota-carrageenan and mixtures thereof, whereby the liquid-gel phase transition is mediated by interaction of the said sulphated polysaccharide derivative with the proteins of the lachrymal fluid.

According to the invention, the liquid-gel phase transition undergone by the ophthalmic composition is a consequence of the interaction of the sulphated polysaccharides in aqueous solution with tear proteins.

The presence of electrolytes further improves the rheological properties of the gels obtained.

This phase transition undergone by the ophthalmic composition increases the contact time of the said composition with the eye, and accordingly enables a sustained or delayed diffusion of the active principle contained in this composition to be obtained.

In addition, it is found that the composition according to the invention leads to larger contents of active principle in the eye as soon as the composition is applied.

Such an ophthalmic composition also possesses the advantages of not spontaneously gelling through the effect of a temperature rise and of possessing a pH close to physiological pH, that is to say of the order of 7.

The sulphated polysaccharides used according to the invention are chosen from kappa-carrageenan, iota-carrageenan and mixtures thereof.

According to a preferred embodiment of the invention, iota-carrageenan will be used.

In an essential feature of the invention, the sulphated polysaccharide derivative is used at a concentration of the order of 0.1 to 5% by weight.

The said composition contains at least one active principle in solution or in suspension in an aqueous medium.

It is possible to use a wide diversity of active principles. The latter may be chosen, in particular, from the following pharmaceutical compounds:

- <u>antibacterial substances</u> such as beta-lactam antibiotics, for example cefoxitin, n-formamidoylthienamycin and other thienamycin derivatives, tetracyclines, chloramphenicol, neomycin, carbenicillin, colistin, penicillin G, polymyxin B, vancomycin cefazolin, cefaloridine, chibrorifamycin, gramicidin, bacitracin and sulphonamides;

  aminoglycoside antibiotics such as gentamicin, kanamycin, amikacin, sisomicin and tobramycin;

  nalidixic acid and its analogues such as norfloxacin and the antimicrobial combination of fluoroalanine/pentizidone, nitrofurazones and their analogues;

- <u>anti-inflammatories</u> such as cortisone, hydrocortisone, hydrocortisone acetate, betamethasone, dexamethasone, dexamethasone sodium phosphate, prednisone, methylprednisolone, medrysone, fluorometholone, prednisolone, triamcinolone, indomethacin, sulindac, its salts and corresponding sulphides, and their analogues;

- <u>miotics and anticholinergics</u> such as echothiopate, pilocarpine, physostigmine salicylate, diisopropyl fluorophosphate, epinephrine, dipivalylepinephrine, neostigmine, echothiopate iodide, demecarium bromide, carbamoylcholine chloride, methacholine, bethanechol and their analogues;

- <u>mydriatics</u> such as atropine, homatropine, scopolamine, hydroxyamphetamine, ephedrine, cocaine, tropicamide, phenylephrine, cyclopentolate, oxyphenonium, eucatropine and their analogues;

- other drugs used in the treatment of eye conditions and lesions, such as:

  antiglaucoma drugs, for example timolol and R-timolol and a combination of timolol or R-timolol with pilocarpine, and also many other adrenergic agonists and/or antagonists; epinephrines and a complex of epinephrine or prodrugs, and dipivefrin derivatives and hyperosmotic agents such as glycerol, mannitol and urea;

- antiparasitic compounds and/or antiprotozoal compounds such as ivermectin, pyrimethamine, trisulphapyrimidine, clindamycin and corticosteroid preparations;
- compounds having antiviral activity such as acyclovir, 5-iodo-2'-deoxyuridine (IDU), adenosine arabinoside (Ara-A), trifluorothymidine, and interferon and interferoninducing agents such as polyI.polyC;
- carbonic anhydrase inhibitors such as acetazolamide, dichlorphenamide, 2-(p-hydroxyphenyl)thio-5-thiophenesulphonamide, 6-hydroxy-2-benzothiazolesulphonamide, 6-pivaloyloxy-2-benzothiazolesulphonamide, MK 927 and MK 417;
- antifungal agents such as amphotericin B, nystatin, flucytosine, natamycin and miconazole;
- anaesthetic agents such as etidocaine, cocaine, benoxinate, dibucaine hydrochloride, dyclonine hydrochloride, naepaine, phenacaine hydrochloride, piperocaine, proparacaine hydrochloride, tetracaine hydrochloride, hexylcaine, bupivacaine, lidocaine, mepivacaine and prilocaine;
- ophthalmic diagnostic agents such as:
    a) those which are used for examining the retina, such as fluorescein sodium;
    b) those which are used for examining the conjunctiva, cornea and lachrymal apparatus, such as fluorescein and rose bengal; and
    c) those which are used for examining abnormal responses of the pupil, such as methacholine, cocaine, adrenaline, atropine, hydroxyamphetamine and pilocarpine;
- ophthalmic agents such as ethylenediaminetetraacetic acid (EDTA) and deferoxamine;
- immunosuppressants and antimetabolites such as methotrexate, cyclophosphamide, 6-mercaptopurine and azathioprine; and antibiotic/anti-inflammatory combinations such as the combination neomycin sulphate/dexamethasone sodium phosphate, and combinations concomitantly treating glaucoma, for example a timolol maleate/aceclidine combination.

The ophthalmic composition according to the invention must naturally fulfil the criteria for use in this field, in particular it should preferably be isotonic and have a pH lying between 5.0 and 8.0. Thus, it may incorporate other components such as tonicity regulators, preservatives and buffer systems.

The present invention also relates to a process for preparing the said composition, characterized in that the active principle and, optionally, the other components are mixed with stirring in an aqueous solution in the presence of 0.1 to 5% by weight of a sulphated polysaccharide derivative selected from kappa-carrageenan, iota-carrageenan and mixtures thereof.

The examples given below will enable other advantages and features of the present invention to be demonstrated.

## EXAMPLE 1 Formulation of an ophthalmic composition

| | |
|---|---|
| Timolol maleate | 0.342 g |
| (corresponds to 0.25 g of timolol base) | |
| Aubygel   (Trade Mark) | 1.500 g |
| Tromethamine | 0.240 g |
| Maleic acid | qs pH 7.0 |
| Mannitol | 3.800 g |
| Benzalkonium chloride | 0.0075 g |
| Water | qs 100.00 g |

Timolol maleate is the active principle, Aubygel X52TA (Trade Mark) marketed by Meso-Rousselot-Satia is iota-carrageenan, the tromethamine/maleic acid combination represents a buffer system, mannitol is a tonicity regulator and benzalkonium chloride a preservative.

## EXAMPLE 2

The composition of Example 1 is tested in vitro according to a gelation test.
In this test, gelation is induced by adding 0.5 ml of the said composition to 1 ml of artificial tears, the composition of which is given in Table I. The formation of the gel is assessed after 30 seconds; incubation at 34°C followed by filtration, by weighing the residue deposited on the filter.

The results are given in Table II.

In protein-free artificial tears, gel formation is small, and may be attributed to an interaction between the carrageenan and the electrolytes present in the artificial tears. The same test carried out on artificial tears containing proteins (lysozyme/bovine albumin fraction V), on the other hand, gives good results. The formation of an opalescent gel having low cohesion is observed. The formation of this gel is, moreover increased by moderate agitation.

## EXAMPLE 3

In order to demonstrate the sustained presence in the eye of the active principle contained in the ophthalmic composition according to the invention, an in vivo study was carried out.

An ophthalmic composition according to the invention and Timoptol (Trade Mark), used by way of comparison, were subjected to a test of ocular penetration of timolol in albino rabbits.

For this purpose, instillations of 50 µl of each of the test solutions were carried out inside the conjunctival sac of living albino rabbits. Following the administration, groups of six animals were then sacrificed at times 10 minutes, 30 minutes, one hour, two hours and four hours, and the timolol assayed in the cornea, aqueous humor and iris.

The timolol concentrations thus assayed are shown, respectively, in Table III for the cornea, Table IV for the aqueous humor and in Table V for the iris and the ciliary bodies.

The results demonstrate that an ophthalmic composition according to the invention gives higher concentrations of active principle in the different tissues than the comparative product Timoptol (Trade Mark) 0.25%, this being the case both at the beginning of the application and after 4 hours.

## TABLE I:

| | | | |
|---|---|---|---|
| • Lysozyme | : | — | 0.15% |
| • Bovine albumin, fraction V | : | — | 0.55% |
| • Sodium chloride | : | 0.67% | 0.67% |
| • Potassium chloride | : | 0.11% | 0.11% |
| • Sodium bicarbonate | : | 0.20% | 0.20% |
| • Calcium chloride | : | 0.008% | 0.008% |
| • Hydrochloric acid | : | qs pH 7.4 | qs pH 7.4 |
| • Demineralized water | : | qs 100.00% | qs 100.00% |

TABLE II:

Gellation test

|  | Weight of the residue | Standard deviation |
|---|---|---|
| · Protein-free artificial tears | 0.219 mg | 0.033 mg |
| · Artificial tears with proteins | 0.514 mg | 0.055 mg |
| · Artificial tears with proteins and agitation | 0.969 mg | 0.075 mg |

The results are expressed on a mean of 12 measurements

## TABLE III

**Timolol concentrations in the cornea of albino rabbits after instillation of formulations containing 0.25% of timolol**

μg of timolol/ml

| Time after administration | 10 mins | 0.5 hr | 1 hr | 2 hr | 4 hr |
|---|---|---|---|---|---|
| TIMOPTOL (Trade Mark) | 17.4 ± 5.5 | 9.2 ± 3.4 | 8.1 ± 3.6 | 2.6 ± 0.8 | 1.3 ± 0.7 |
| Ophthalmic composition according to the invention (Example 1) | 60.0 ± 14.8* | 64.0 ± 22.0* | 41.9 ± 14.5* | 15.6 ± 9.5* | 2.9 ± 1.9* |

Each result represents the mean ± the standard deviation of measurements carried out on 12 eyes

\* Values significantly different ($p < 0.005$) from those obtained with Timoptol (Trade Mark)

(Student's t test)

EP 0 424 043 B1

**TABLE IV**

Timolol concentrations in the aqueous humor of albino rabbits after instillation
of formulations containing 0.25% of timolol

$\mu$g of timolol/ml

| Time after administration | 10 mins | 0.5 hr | 1 hr | 2 hr | 4 hr |
|---|---|---|---|---|---|
| TIMOPTOL (Trade Mark) | 0.5 ± 0.2 | 0.7 ± 0.2 | 0.8 ± 0.5 | 0.3 ± 0.1 | 0.1 ± 0.1 |
| Ophthalmic composition according to the invention (Example 1) | 0.9 ± 0.4* | 3.1 ± 0.9* | 3.5 ± 0.9* | 1.5 ± 0.5* | 0.2 ± 0.1 |

Each result represents the mean ± the standard deviation of measurements carried out on 12 eyes

* Values significantly different (p < 0.005) from those obtained with Timoptol (Trade Mark) (Student's t test)

**TABLE V**

Timolol concentrations in the iris and the ciliary bodies of albino rabbits after instillation of formulations containing 0.25% of timolol

$\mu$g of timolol/ml

| Time after administration | 10 mins | 0.5 hr | 1 hr | 2 hr | 4 hr |
|---|---|---|---|---|---|
| TIMOPTOL (Trade Mark) | 1.2 ± 0.4 | 1.2 ± 0.4 | 1.3 ± 0.4 | 0.4 ± 0.2 | 0.2 ± 0.1 |
| Ophthalmic composition according to the invention (Example 1) | 3.8 ± 1.6* | 4.1 ± 0.9* | 4.0 ± 1.6* | 1.7 ± 0.8* | < 0.4 |

Each result represents the mean ± the standard deviation of measurements carried out on 12 eyes

* Values significantly different (p < 0.005) from those obtained with Timoptol (Trade Mark) (Student's t test)

## Claims

### Claims for the following Contracting States : AT, BE, CH, DE, DK, FR, GB, IT, LI, LU, NL, SE

1. A liquid ophthalmic composition which undergoes liquid-gel phase transition upon administration to the eye and comprises an aqueous solution of at least one active principle, characterized in that the composition contains 0.1 to 5% by weight of a sulphated polysaccharide derivative selected from kappa-carrageenan, iota-carrageenan and mixtures thereof, whereby the liquid-gel phase transition is mediated by interaction of the said sulphated polysaccharide derivative with the proteins of the lachrymal fluid.

2. An ophthalmic composition according to Claim 1, characterized in that it has a pH close to physiological pH.

3. An ophthalmic composition according to Claim 1 or Claim 2, characterized in that the sulphated polysaccharide is iota-carrageenan.

4. An ophthalmic composition according to any one of the preceding Claims, characterized in that it contains other compounds chosen from tonicity regulators, preservatives and buffer systems.

5. An ophthalmic composition according to any one of the preceding Claims, characterized in that the active principle is chosen from antiglaucoma agents, antibiotics and antiviral agents.

6. An ophthalmic composition according to Claim 5, characterized in that the active principle is timolol.

7. A process for preparing an ophthalmic composition according to any one of the preceding Claims, characterized in that the active principle and, optionally, the other components are mixed with stirring in an aqueous solution in the presence of 0.1 to 5% by weight of a sulphated polysaccharide derivative selected from kappa-carrageenan, iota-carrageenan and mixtures thereof.

### Claims for the following Contracting State : ES

1. A process for preparing a liquid ophthalmic composition which undergoes liquid-gel phase transition upon administration to the eye, characterized in that it comprises mixing an aqueous solution of at least one active principle with 0.1 to 5% by weight of a sulphated polysaccharide derivative selected from kappa-carrageenan, iota-carrageenan and mixtures thereof, whereby the liquid-gel phase transition is mediated by interaction of the said sulphated polysaccharide derivative with the proteins of the lachrymal fluid.

2. A process according to Claim 1, characterized in that the composition prepared has a pH close to physiological pH.

3. A process according to Claim 1 or Claim 2, characterized in that the sulphated polysaccharide is iota-carrageenan.

4. A process according to any one of the preceding Claims, characterized in that the composition is prepared in such a way that it contains other compounds chosen from tonicity regulators, preservatives and buffer systems.

5. A process according to any one of the preceding Claims, characterized in that the active principle is chosen from antiglaucoma agents, antibiotics and antiviral agents.

6. A process according to Claim 5, characterized in that the active principle is timolol.

## Patentansprüche

### Patentansprüche für folgende Vertragsstaaten : AT, BE, CH, DE, DK, FR, GB, IT, LI, LU, NL, SE

1. Flüssiges Augenpräparat, das bei Anwendung am Auge einen Flüssigkeit-Gel-Phasenübergang durchmacht und eine wäßrige Lösung aus mindestens einem Wirkprinzip enthält, dadurch gekennzeichnet, daß das Präparat 0,1 bis 5 Gew.-% eines sulfathaltigen Polysaccharid-Derivates enthält, ausgewählt aus kappa-Carrageenan, iota-Carrageenan und Gemischen davon, wobei der Flüssigkeit-Gel-Phasenübergang durch eine Wechselwirkung des genannten sulfathaltigen Polysaccarid-Derivates mit den Proteinen der Tränenflüssigkeit ausgelöst wird.

2. Augenpräparat nach Anspruch 1, dadurch gekennzeichnet, daß es einen pH-Wert in der Nähe des physiologischen pH-Wertes aufweist.

3. Augenpräparat nach Anspruch 1 oder 2, dadurch gekennzeichnet, daß das sulfathaltige Polysaccharid iota-Carrageenan ist.

4. Augenpräparat nach einem der vorhergehenden Ansprüche, dadurch gekennzeichnet, daß es weitere Verbindungen, ausgewählt aus Tonus-Regulatoren, Konservierungsstoffen und Puffersystemen, enthält.

5. Augenpräparat nach einem der vorausgegangenen Ansprüche, dadurch gekennzeichnet, daß das Wirkprinzip aus Antiglaucom-Mitteln, Antibiotika und antiviralen Mitteln ausgewählt ist.

6. Augenpräparat nach Anspruch 5, dadurch gekennzeichnet, daß das Wirkprinzip Timolol ist.

7. Verfahren zur Herstellung eines Augenpräparates nach einem der vorausgegangenen Ansprüche, dadurch gekennzeichnet, daß das Wirkprinzip und gegebenenfalls die anderen Komponenten unter Rühren in einer wäßrigen Lösung in Gegenwart von 0,1 bis 5 Gew.-% eines sulfathaltigen Polysaccharid-Derivates, ausgewählt aus Kappa-Carrageenan, iota-Carrageenan und Gemischen davon, vermischt werden.

**Patentansprüche für folgenden Vertragsstaat : ES**

1. Verfahren zur Herstellung eines flüssigen Augenpräparates, das bei Anwendung am Auge einen Flüssigkeit-Gel-Phasenübergang durchmacht, dadurch gekennzeichnet, daß es umfaßt das Vermischen einer wäßrigen Lösung aus mindestens einem Wirkprinzip mit 0,1 bis 5 Gew.-% eines sulfathaltigen Polysaccharid-Derivates, ausgewählt aus kappa-Carrageenan, iota-Carrageenan und Gemischen davon, wobei der Flüssigkeit-Gel-Phasenübergang durch Wechselwirkung des genannten sulfathaltigen Polysaccharid-Derivates mit den Proteinen der Tränenflüssigkeit ausgelöst wird.

2. Verfahren nach Anspruch 1, dadurch gekennzeichnet, daß das hergestellte Präparat eine pH-Wert in der Nähe des physiologischen pH-Wertes aufweist.

3. Verfahren nach Anspruch 1 oder 2, dadurch gekennzeichnet, daß das sulfathaltige Polysaccharid iota-Carrageenan ist.

4. Verfahren nach einem der vorausgegangenen Ansprüche, dadurch gekennzeichnet, daß das Präparat so hergestellt wird, daß es weitere Verbindungen, ausgewählt aus Tonus-Regulatoren, Konservierungsstoffen und Puffersystemen, enthält.

5. Verfahren nach einem der vorausgegangenen Ansprüche, dadurch gekennzeichnet, daß das Wirkprinzip aus Antiglaucom-Mitteln, Antibiotika und antiviralen Mitteln ausgewählt wird.

6. Verfahren nach Anspruch 5, dadurch gekennzeichnet, daß das Wirkpinzip Timolol ist.

**Revendications**

**Revendications pour les Etats contractants suivants : AT, BE, CH, DE, DK, FR, GB, IT, LI, LU, NL, SE**

1. Composition ophtalmique liquide qui présente une transition de phase liquide-gel lors de l'administration à l'oeil et qui comprend une solution aqueuse d'au moins un principe actif, la composition ètant caractérisée en ce qu'elle contient 0,1 à 5 % en poids d'un polysaccharide sulfaté choisi parmi la kappa-carragénine, l'iota-carragénine et leurs mélanges, la transition de phase liquide-gel s'effectuant par l'intermédiaire d'une interaction dudit polysaccharide sulfaté avec les protéines du liquide lacrymal.

**2.** Composition ophtalmique selon la revendication 1, caractérisée en ce qu'elle a un pH proche du pH physiologique.

**3.** Composition ophtalmique selon la revendication 1 ou la revendication 2, caractérisée en ce que le polysaccharide sulfaté est l'iota-carragénine.

**4.** Composition ophtalmique selon l'une quelconque des revendications précédentes, caractérisée en ce qu'elle contient d'autres composés choisis parmi les régulateurs de la tonicité, les conservateurs et les systèmes tampons.

**5.** Composition ophtalmique selon l'une quelconque des revendications précédentes, caractérisée en ce que le principe actif est choisi parmi les agents antiglaucomateux, les antibiotiques et les agents antiviraux.

**6.** Composition ophtalmique selon la revendication 5, caractérisée en ce que le principe actif est le timolol.

**7.** Procédé pour préparer une composition ophtalmique selon l'une quelconque des revendications précédentes, caractérisé en ce que l'on mélange, avec agitation en solution aqueuse, le principe actif et, le cas échéant, les autres compoants, en présence de 0,1 à 5 % en poids d'un polysaccharide sulfaté choisi parmi la kappa-carragénine, l'iota-carragénine et leurs mélanges.

**Revendications pour l'Etat contractant suivant : ES**

**1.** Procédé pour préparer une composition ophtalmique liquide qui présente une transition de phase liquide-gel lors de l'administration à l'oeil, caractérisé en ce qu'il comprend le mélange, en solution aqueuse, d'au moins un principe actif avec 0,1 à 5 % en poids d'un polysaccharide sulfaté choisi parmi la kappa-carragénine, l'iota-carragénine et leurs mélanges, la transition de phase liquide-gel s'effectuant par l'intermédiaire d'une interaction dudit polysaccharide sulfaté avec les protéines du liquide lacrymal.

**2.** Procédé selon la revendication 1, caractérisé en ce que la composition préparée a un pH proche du pH physiologique.

**3.** Procédé selon la revendication 1 ou la revendication 2, caractérisé en ce que le polysaccharide sulfaté est l'iota-carragénine.

**4.** Procédé selon l'une quelconque des revendications précédentes, caractérisé en ce que la composition est préparée de telle sorte qu'elle contient d'autres composés choisis parmi les régulateurs de la tonicité, les conservateurs et les systèmes tampons.

**5.** Procédé selon l'une quelconque des revendications précédentes, caractérisé en ce que le principe actif est choisi parmi les agents antiglaucomateux, les antibiotiques et les agents antiviraux.

**6.** Procédé selon la revendication 5, caractérisé en ce que le principe actif est le timolol.